(19) Europäisches Patentamt
European Patent Office
Office européen des brevets

(11) Numéro de publication : 0 601 897 A1

(12) **DEMANDE DE BREVET EUROPEEN**

(21) Numéro de dépôt : **93402053.8**

(22) Date de dépôt : **13.08.93**

(51) Int. Cl.$^5$ : **C07H 5/06,** C07H 13/04, C07H 13/06

(30) Priorité : **28.08.92 FR 9210347**

(43) Date de publication de la demande : **15.06.94 Bulletin 94/24**

(84) Etats contractants désignés : **BE DE GB IT NL**

(71) Demandeur : **ERIDANIA BEGHIN-SAY**
**F-59239 Thumeries (FR)**

(72) Inventeur : **Lubineau, André**
**2, allée des Près,**
**Hameau de Rouillon**
**F-91410 Dourdan (FR)**
Inventeur : **Drouillat, Bruno**
**27, rue des Meuniers**
**F-75012 Paris (FR)**
Inventeur : **Auge, Jacques**
**16, allée du Pré Lambesc**
**F-91190 Gif-Sur-Yvette (FR)**
Inventeur : **Mentech, Julio**
**1D, rue Phélypeaux**
**F-69100 Villeurbanne (FR)**

(74) Mandataire : **David, Daniel et al**
**KAYSERBERG**
**Service Propriété Industrielle**
**23 boulevard Georges Clemenceau**
**F-92402 Courbevoie Cédex (FR)**

(54) **Procécé de préparation de glycosylamines à partir de sucres réducteurs.**

(57) L'invention concerne un nouveau procédé de préparation de glycosylamines à partir de sucres réducteurs.

Ce procédé consiste à faire réagir le sucre réducteur avec une solution contenant de l'ammoniaque et du bicarbonate d'ammonium.

L'invention concerne également l'utilisation des glycosylamines formées comme intermédiaires de synthèse.

EP 0 601 897 A1

L'invention concerne un nouveau procédé de préparation de glycosylamines à partir de sucres réducteurs.

La glucosylamine, de même que les glycosylamines d'une façon générale, sont connues comme intermédiaires de synthèse par exemple pour la préparation des amides correspondants, ainsi que pour la préparation de divers produits d'acylation particulièrement intéressants dans la préparation de produits à activité détergente.

Le développement de ces produits s'est trouvé limité du fait du manque de stabilité de l'intermédiaire de synthèse et de la difficulté à l'obtenir de façon économique et quantitative.

En effet il est connu que les produits de la classe des glycosylamines présentent de considérables différences de stabilité mais ne sont d'une façon générale pas particulièrement stables. La glucosylamine, en particulier, s'hydrolyse facilement en glucose, très rapidement entre pH 3 et 7 ; d'autre part, elle peut former en solution une bis-glucosylamine par élimination d'ammoniac. Même cristalline, il vaut mieux éviter de la conserver dans un dessiccateur contenant un desséchant tel que $P_2O_5$.

Lorsque l'on veut synthétiser de la glucosylamine, la première idée qui vient à l'esprit est de faire réagir une solution aqueuse d'ammoniac sur du glucose selon le mécanisme suivant :

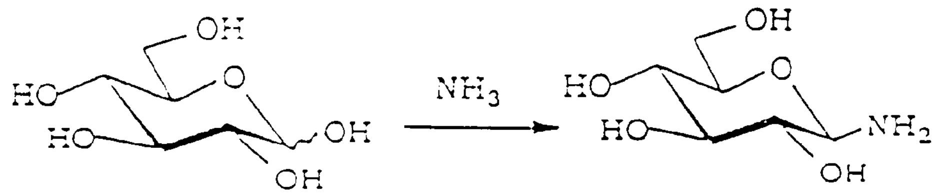


Une telle réaction ainsi que les produits de réaction des sucres avec l'ammoniac en solution aqueuse est abondamment étudiée dans "Reactions of free sugars with aqueous ammoniac" par M.J. KORT, Adv. Carb. Chem. Biochem, 25, 1970, 311-349 d'où il ressort qu'un grand nombre de sous-produits peuvent être obtenus lors de la réaction du fait de réactions annexes d'épimérisation, réarrangements de type Amadori, dégradations et condensations diverses.

Il apparait donc difficile d'obtenir de façon simple une glycosylamine pure en solution à partir de sucres réducteurs et d'ammoniaque.

Par ailleurs, en vue d'éviter les phénomènes d'hydrolyse on a décrit de façon déja très ancienne des procédés recourant à de l'ammoniac dissous dans un solvant organique. Ainsi, on a décrit dans Rec. Trav. Chim. Pays-Bas, 12 (1895), 98-105, un procédé consistant à dissoudre du glucose dans une solution de méthanol saturée d'ammoniac, et à attendre la cristallisation de la glucosylamine à 0°C ; celle-ci dure plusieurs mois avec un rendement de 15%.

D'après H.S. Isbel et H.L. Frush, J. Org. Chem., 23 (1958) 1309-1319, le rendement de la réaction peut etre amélioré en dissolvant le glucose dans une solution saturée de chlorure d'ammonium dans le méthanol. Cette méthode peu efficace a été malgré tout étendue à un grand nombre de glycosylamines, telles que galactosylamine, mannosylamine.

Plus récemment, L.M. Likhosherstov et al., Carbohydr. Res. 146 (1986) C1-C5 puis E. Kallin et al., J. Carbohydr. Chem., 8 (1989) 597-611 ont préparé respectivement des 2-acétamido-2-déoxyglucosylamines et la lactosylamine en dissolvant le sucre libre correspondant dans une solution aqueuse saturée de bicarbonate d'ammonium.

La réaction dure de 3 à 45 jours à température ambiante. D'autre part, l'isolement de la glycosylamine après passage sur résine acide puis basique est rendu difficile du fait de sa fragilité et du fort excès de bicarbonate d'ammonium ; les rendements restent donc modestes.

En effet, les mécanismes mis en oeuvre au cours de la réaction du glucose sur le bicarbonate d'ammonium sont les suivants :

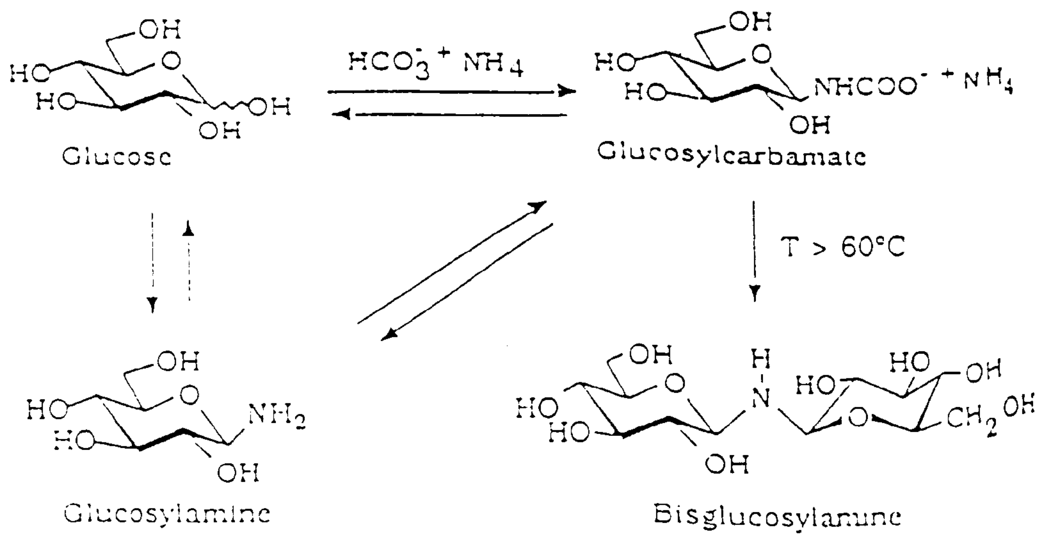


Glucose

Glucosylcarbamate

Glucosylamine

Bisglucosylamine

Kallin et al. ont montré que le lactose donne accès à la lactosylamine en présence d'une solution saturée de bicarbonate d'ammonium à température ambiante. Ils ont montré qu'il se forme dans les conditions opératoires un carbamate qui, au cours du traitement sur résine pour éliminer le sel, se décompose en lactosylamine.

Des essais réalisés par la demanderesse ont montré que le même phénomène se produisait avec le glucose conduisant à la formation de glucosylcarbamate.

De plus, la demanderesse a observé qu'à partir de 60°C il y avait condensation de deux molécules de glycosylcarbamate pour donner de la bis-glucosylamine avec élimination d'ammoniac.

Ainsi donc, il n'existe pas à l'heure actuelle de procédé permettant de préparer la glucosylamine de façon quantitative en solution aqueuse et permettant par conséquent de l'utiliser in situ sans purification comme intermédiaire de synthèse, en particulier dans des réactions d'acylation.

En effet, l'étude des documents de littérature cités précédemment ainsi que les essais réalisés par la demanderesse montrent que la réaction du glucose avec des solutions aqueuses d'ammoniac conduit à la formation de mélanges contenant de nombreuses impuretés mal définies à côté de la glucosylamine alors que la réaction du bicarbonate d'ammonium ne peut conduire de façon quantitative à la formation de glucosylamine qu'à condition d'utiliser un très large excès de bicarbonate, de l'ordre de 35 pour 1 glucose.

La demanderesse a maintenant découvert un nouveau procédé permettant de former directement en solution aqueuse la glucosylamine ainsi que différentes glycosylamines à partir des sucres réducteurs correspondants sans impuretés et dans des conditions économiquement acceptables.

Le procédé mis au point par la demanderesse présente l'avantage de n'utiliser que des réactifs peu coûteux, à des concentrations économiquement acceptables et de permettre la formation quantitative de glycosylamines à partir de sucres réducteurs. Le fait que ces glycosylamines soient obtenues de façon quantitative et sans impuretés en solution aqueuse permet d'envisager leur utilisation in situ dans Le milieu et sans purification ultérieure comme intermédiaires de synthèse, notamment dans des réactions d'acylation.

Ainsi, selon une de ses caractéristiques essentielles, l'invention concerne un procédé de préparation de glycosylamines à partir de sucres réducteurs en milieu aqueux, caractérisé en ce que l'on fait réagir lesdits sucres réducteurs avec une solution contenant de l'ammoniaque et du bicarbonate d'ammonium.

Selon une variante avantageuse l'ammoniaque est en excès par rapport à la quantité stoechiométrique nécessaire pour réaliser la réaction.

Selon une caractéristique de l'invention, le bicarbonate d'ammonium est introduit en quantité suffisante pour obtenir la glycosylamine sans impureté, alors qu'il ressort de l'art antérieur, ainsi que des essais réalisés par la demanderesse, que l'ammoniac en solution seul conduit par réaction avec les sels réducteurs à des glycosylamines contenant une quantité non négligeable d'impuretés mal définies.

Un avantage du procédé selon l'invention est qu'il permet par ailleurs d'utiliser des quantités beaucoup plus faibles de bicarbonate d'ammonium que celles utilisées en absence d'ammoniaque.

Selon une caractéristique de l'invention, le sucre réducteur utilisé est le glucose.

Selon d'autres caractéristiques de l'invention, le sucre réducteur peut être un autre saccharide, par exemple le galactose ou un disaccharide, par exemple le lactose, le cellobiose, le maltose.

La réaction est réalisée en milieu aqueux en présence d'un excès d'ammoniaque. La solution d'ammoniaque utilisée sera avantageusement une solution commerciale à 16M.

La quantité de bicarbonate d'ammonium est déterminée de façon à obtenir, en présence d'une solution aqueuse de concentration donnée en glucose ou en sucre réducteur et d'un excès d'ammoniaque, une glucosylamine ou une glycosylamine sans impureté.

Ainsi, par exemple, pour une solution de glucose à 0,2M en présence de $NH_3$ aqueux à 16 M à 42°C, au bout de 24 h les résultats consignés dans le tableau 1 ci-dessous ont été observés en fonction de la concentration du bicarbonate d'ammonium introduit.

<u>Tableau 1</u>

| $NH_4^+, HCO_3^-$ | Amine | Carbamate | Glucose | Impuretés |
|---|---|---|---|---|
| 0M | 51% | 0% | 22% | 27% |
| 0,05M | 56% | 0% | 31% | 13% |
| 0,10M | 65% | 0% | 35% | 0% |
| 0,20M | 78% | 8% | 14% | 0% |
| 0,40M | 80% | 10% | 10% | 0% |
| 1M | 90% | 10% | 0% | 0% |

En 36 h, la réaction avec du glucose 0,2M et du bicarbonate d'ammonium 0,2M a donné quantitativement de la β-glucosylamine.

Par ailleurs la demanderesse à cherché à augmenter la concentration en glucose tout en Limitant celle de bicarbonate d'ammonium.

Les tableaux 2 et 3 ci-dessous donnent les résultats obtenus :

Tableau 2

Conditions

glucose 0,4M

$NH_3$ aqueux 16M

température 42°C

| $NH_4^+, HCO_3^-$ | Temps | 18 h | 24 h | 36 h |
|---|---|---|---|---|
| 0,2M | Glucosylamine<br>Glucose | 83%<br>17% | 93%<br>7% | 100%<br>0% |
| 0,4M | Glucosylamine<br>Glucose | 100%<br>0% | | |

Tableau 3

Conditions

glucose 0,6M

$NH_3$ aqueux 16M

température 42°C

| $NH_4^+, HCO_3^-$ | Espèce | Temps = 18 h |
|---|---|---|
| 0,2M | Glucosylamine<br>Glucose<br>Bis-glucosylamine | 77%<br>15%<br>8% |

Tableau 3 (suite)

| $NH_4^+, HCO_3^-$ | Espèce | Temps = 18 h |
|---|---|---|
| 0,4M | Glucosylamine<br>Glucose<br>Bis-glucosylamine | 95%<br>0%<br>5% |

Si on cherche à réduire au maximum la concentration en bicarbonate d'ammonium (pour une question de coût d'une part et pour faciliter son éLimination dans une phase ultérieure), la concentration 0,6M en glucose

se trouve trop élevée, car on forme de la bis-glucosylamine avant la fin de la réaction ; par contre, on peut opérer à 0,4M de glucose. Dans ce cas, la réaction dure 36 h à 42°C en présence d'une solution aqueuse 16M d'ammoniac et 0,2M de bicarbonate d'ammonium.

Ainsi, donc la demandresse a mis en évidence que les conditions sont avantageusement les suivantes pour préparer la glucosylamine quantitativement à partir du glucose sans impuretés visibles en RMN : 0,2M-0,4M de glucose ; 0,2M de bicarbonate d'ammonium ; ammoniac 16M dans l'eau ; 36 h à 42°C.

Ces conditions permettent de lyophiliser simplement la glucosylamine pour la soumettre à une réaction ultérieure par exemple d'acylation.

Bien entendu, l'homme du métier sait que ces conditions ne sont nullement limitatives et qu'en particulier les concentrations optimales de chacun des réactifs varient avec la température.

La température dans le cas du glucose sera avantageusement choisie entre 20 et 60°C, par exemple 42°C.

La méthode exposée précédemment peut être utilisée avec tous les sucres réducteurs en particulier avec les monosaccharides comme le galactose ou les disaccharides comme le lactose, le cellobiose, le maltose.

Les conditions opératoires sont avantageusement les suivantes :

| Conditions | |
|---|---|
| mono- ou disaccharide | 0,2M |
| bicarbonate d'ammonium | 0,2M |
| $NH_3$ | 16M |
| température | 42°C |
| temps de réaction | 36 h |

Un avantage particulier du procédé de l'invention est qu'il permet une synthèse quantitative des glycosylamines à partir des sucres réducteurs correspondants et permet ainsi une utilisation aisée sans isolation intermédiaire de la glycosylamine en particulier comme intermédiaire dans des réactions mettant en oeuvre un produit électrophile, tout particulièrement des réactions d'acylation.

Selon un autre aspect de l'invention, elle concerne donc l'utilisation de la glycosylamine directement obtenue dans le procédé selon l'invention pour réaliser des réactions mettant en oeuvre un produit électrophile, tout particulièrement des réactions d'acylation.

A titre d'exemples de réactions mettant en oeuvre un électrophile, on citera les réactions avec les chlorures d'acide, les anhydrides, les aldéhydes et les cétones, les époxydes, les chlorocarbonates.

A titre d'exemples de réactions d'acylation, on citera les réactions de peracétylation ainsi que les réactions d'acylation sélective sur l'atome d'azote (N-acylation).

La réaction de peracétylation est avantageusement effectuée sur la glycosylamine préalablement lyophilisée par mise en contact de cette glycosylamine avec un anhydride en milieu solvant.

A titre d'exemple de réaction de peracylation, on citera la peracétylation de la glucosylamine. Cette réaction est avantageusement réalisée sur la glucosylamine préparée selon le procédé de l'invention puis lyophilisée et mise en contact à froid, de préférence vers 0°C, avec un mélange d'anhydride acétique et de pyridine ; le schéma de réaction étant le suivant :

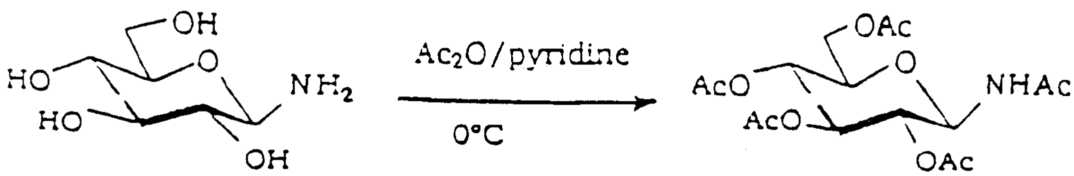


Dans ces conditions la glucosylamine peracétylée est formée avec un rendement de 77%.

A titre d'exemple de réaction d'acylation sélective sur l'atome d'azote on citera la réaction de la glucosylamine en solution dans un solvant tel que l'éthanol sur un anhydride, par exemple l'anhydride octanoïque selon le schéma suivant :

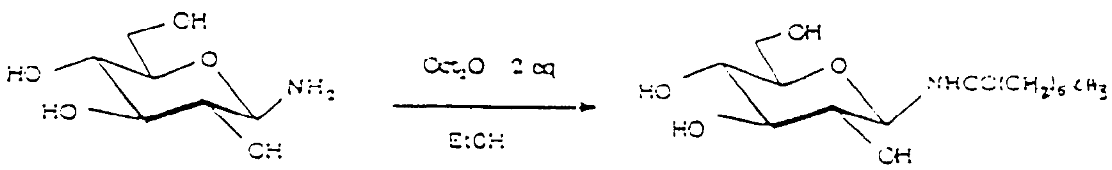


Dans ces conditions, la glucosylamine N-octanoylée est formée avec un rendement de 82%.

Pour débarrasser le produit ci-dessus des sels il est avantageusement acétylé, par exemple avec un mélange anhydride acétique-pyridine, pour donner l'acétate ci-dessous :

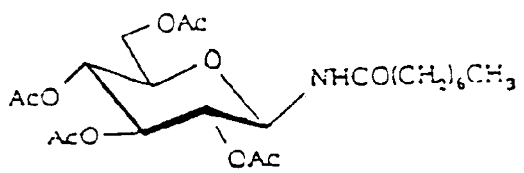

qui par déacétylation conduit à la glucosylamine N-octanoylée qui pourra à son tour être purifiée ultérieurement, par exemple par chromatographie sur colonne.

**Exemples**

Les produits préparés ont été contrôlés par RMN du proton et du $^{13}C$.

Les produits dissous dans $CDCl_3$ ont pour référence interne le TMS (δ0).

Les produits dissous dans $D_2O$ ont pour référence interne l'acétone (δ2.09 en $^1H$ ; δ30.50 en $^{13}C$).

Exemple 1 Préparation de la N-acétyl-2,3,4,6-tétra-O-acétyl-β-D-glucopyranosylamine (dénommée ci-dessous A)

On dissout 1 g de glucose (0,2M) et 0,439 g de bicarbonate d'ammonium (0,2M) dans 27 ml d'ammoniaque 16M. Cette solution est chauffée 36 h à 42°C, concentrée au tiers du volume initial, puis lyophilisée. On vérifie la présence de glycosylamine en RMN $^1H$. La glycosylamine est dissoute dans 13,6 ml de pyridine. On ajoute lentement 9,5 ml d'anhydride acétique à 0°C. La solution est laissée une nuit à température ambiante. Elle est ensuite versée dans 100 ml d'acide chlorhydrique aqueux 0,1M. Le produit est extrait avec 100 ml de dichlorométhane et on neutralise avec 100 ml d'eau saturée de bicarbonate de sodium. Le dichlorométhane est évaporé à sec. Le résidu est recristallisé dans l'éthanol. On obtient 1,665 g de produit A (rdt = 77%). F = 161°C.$[\alpha]_D^{20}$ = 17° (c 0,97, $CHCl_3$).

Exemple 2 Préparation de la N-octanoyl-2,3,4,6-tétra-O-acétyl-β-D-glucosylamine (ci-après dénommée produit B)

On dissout 1 g de glucose (0,2M) et 0,439 g de bicarbonate d'ammonium (0,2M) dans 27 ml d'ammoniaque 16M. Cette solution est chauffée 36 h à 42°C, concentrée au tiers du volume initial, puis lyophilisée. La glycosylamine est dissoute dans 50 ml d'éthanol. On ajoute 3,7 ml d'anhydride octanoïque (2 équivalents) à température ambiante. Après 10 min, on évapore l'éthanol. On reprend avec 100 ml d'eau, puis on extrait l'acide octanoique avec 2 fois 100 ml d'éther. L'eau est évaporée. On ajoute 13,6 ml de pyridine et on ajoute Lentement 9,5 ml d'anhydride acétique à 0°C. La solution est Laissée une nuit à température ambiante. Elle est ensuite versée dans 100 ml d'acide chlorhydrique aqueux 0,1M. Le produit est extrait avec 100 ml de dichlorométhane et on neutralise avec 100 ml d'eau saturée de bicarbonate de sodium. Le dichlorométhane est évaporé à sec. Le produit est chromatographié sur silice 20-45 μm avec un mélange hexane/éther 1/3. On obtient 2,10 g de produit B (rdt 82%).

Exemple 3 Préparation de la N-octanoyl-β-D-glucopyranosylamine (dénommée ci-dessous C)

0,570 g de produit B de l'exemple 2 est dissous dans une solution de méthylate de sodium 0,1 mM. La réaction est immédiate. La solution est passée sur 60 g de résine Dowex 50 X8-100 sous forme protonée ; la

résine est lavée avec 100 ml de méthanol qui est ensuite évaporé. Le produit est dissous dans 30 ml d'eau et passé sur 25 g de gel Bio-Beads SM 2. Le gel est lavé avec 100 ml d'eau. Le produit est élué avec 30 ml de méthanol. Après évaporation du méthanol, on obtient 0,334 g de produit C (rdt 91%).

## Revendications

1. Procédé de préparation de glycosylamines à partir de sucres réducteurs, caractérisé en ce que l'on fait réagir lesdits sucres réducteurs avec une solution contenant de l'ammoniaque et du bicarbonate d'ammonium.

2. Procédé selon la revendication 1, caractérisé en ce que l'ammoniaque est en excès par rapport à la quantité stoechiométrique.

3. Procédé selon l'une des revendications 1 ou 2, caractérisé en ce que le bicarbonate d'ammonium se trouve en quantité suffisante pour obtenir une transformation quantitative du sucre réducteur en glycosylamine.

4. Procédé selon l'une des revendications 1 à 3, caractérisé en ce que le sucre réducteur est le glucose.

5. Procédé selon l'une des revendications 1 à 3, caractérisé en ce que le sucre réducteur est un monosaccharide ou un disaccharide.

6. Utilisation des glycosylamines issues du procédé selon l'une des revendications 1 à 5, dans des réactions mettant en oeuvre un produit électrophile, en particulier dans des réactions d'acylation.

7. Utilisation selon la revendication 6, caractérisée en ce que la réaction d'acylation est une réaction de peracétylation ou d'acylation sélective, par exemple de N-acylation.

Office européen des brevets

# RAPPORT DE RECHERCHE EUROPEENNE

Numero de la demande
EP 93 40 2053

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 413 675 (MONSANTO COMPANY)<br>* page 7, ligne 35 - page 15, ligne 50 *<br>--- | 1-7 | C07H5/06<br>C07H13/04<br>C07H13/06 |
| A | WO-A-90 10023 (BIOCARB AB)<br>* page 9, ligne 10 - page 11, ligne 7 *<br>--- | 1-7 | |
| A | US-A-4 638 816 (COX R.H. ET AL)<br>* colonne 6, ligne 21 - ligne 46 *<br>--- | 1 | |
| A | JOURNAL OF ORGANIC CHEMISTRY<br>vol. 28 , Octobre 1963 , EASTON US<br>pages 2784 - 2789<br>HODGE J.E. AND MOY B.F. 'Preparation and properties of Dialditylamines'<br>* page 2786, colonne 2; tableau I *<br>--- | 1 | |
| D,A | CARBOHYDRATE RESEARCH.<br>vol. 146 , 1986 , AMSTERDAM NL<br>pages C1 - C5<br>LIKHOSHERTSOV L.M. ET AL 'A new simple synthesis of amino sugar .beta.-D-glycosylamines'<br>* le document en entier *<br>----- | 1 | DOMAINES TECHNIQUES RECHERCHES (Int.Cl.5)<br>C07H |

Le présent rapport a été établi pour toutes les revendications

| Lieu de la recherche | Date d'achèvement de la recherche | Examinateur |
|---|---|---|
| LA HAYE | 6 Janvier 1994 | Day, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)